# EUROPEAN PATENT APPLICATION

(11) **EP 4 163 923 A1**
(43) Date of publication of application: **12.04.2023**
(21) Application number: 22182515.1
(22) Date of filing: 01.07.2022
(51) Int. Cl.: G16H 10/60, G16H 10/65

(54) **VACCINATION DATA PRESENTATION METHOD, VACCINATION DATA PRESENTATION SYSTEM AND VACCINATION DATA AUTHENTICATION SERVER**

(30) Priority: 06.10.2021 TW 110137244
(71) Applicant: AI Bioelectronic Healthtech Co., Ltd., 32450 Taoyuan City (TW)
(72) Inventor: HO, Yen-Yi, 32450 Taoyuan City (TW); HUANG, Yen-Yun, 32450 Taoyuan City (TW)
(74) Representative: Viering, Jentschura & Partner mbB Patent- und Rechtsanwälte

(57) **Abstract**

A vaccination data presentation method applied to an electronic device (900) for presenting a vaccination data is disclosed. The vaccination data presentation method includes: receiving a vaccination data (E) and an identification data (F); storing the vaccination data (E) and the identification data (F); generating a first data presentation page (A) according to the vaccination data (E) and the identification data (F), and displaying the first data presentation page (A) on the electronic device (900), wherein the first data presentation page (A) displays the vaccination data (E) and the identification data (F) and includes a data privacy button (C); if the data privacy button (C) is triggered, generating a second data presentation page (A1) to replace the first data presentation page (A), wherein the second data presentation page (A1) displays the vaccination data (E) and does not display the identification data (F).

## Description

### BACKGROUND OF THE INVENTION

### 1. Field of the Invention

The present invention relates to a vaccination data presentation method, a vaccination data presentation system and a vaccination data authentication server; more particularly, the present invention relates to a vaccination data presentation method, a vaccination data presentation system and a vaccination data authentication server which can easily and conveniently provide the vaccination data of individuals and ensure the security of personal information.

### 2. Description of the Related Art

In recent years, due to the global spread of the Coronavirus Disease 2019 (COVID-19) pandemic, many pharmaceutical companies have produced coronavirus vaccines. These vaccines can produce antibodies in the human body, which can reduce the risk of serious illness or death. In order to reduce the impact of the epidemic, some businesses, restaurants or exhibitions will allow only people who have been vaccinated to enter, and those who have been vaccinated must also provide proof of vaccination to prove that they have resistance to the disease.

However, the general document providing proof of vaccination, referred to henceforth as the vaccination card, is made of paper, and it can easily be damaged or lost. People who have been vaccinated but do not have their vaccination cards on their persons may be unable to enter places where their health status must be verified before entry. In addition, detailed personal information is recorded on the vaccination card; thus, when the vaccination card is presented to others, such information may be secretly recorded by unscrupulous persons and used for illegal purposes.

Therefore, there is a need to provide a new vaccine data presentation method and device which can easily and conveniently provide the vaccination data of individuals while ensuring the security of personal information.

### SUMMARY OF THE INVENTION

It is an object of the present invention to provide a vaccination data presentation method which can easily and conveniently provide the vaccination data of individuals and ensure the security of personal information.

To achieve the abovementioned object, a vaccination data presentation method of the present invention is applied to an electronic device for displaying a vaccination data. The vaccination data presentation method includes: receiving the vaccination data and an identification data; storing the vaccination data and the identification data; according to the vaccination data and the identification data, generating a first data presentation page and displaying the first data presentation page on the electronic device, wherein the first data presentation page displays the vaccination data and the identification data and further includes a data privacy button; if the data privacy button is triggered, generating a second data presentation page to replace the first data presentation page, wherein the second data presentation page displays the vaccination data and does not display the identification data.

According to one embodiment of the present invention, the first data presentation page and the second data presentation page each include a QR code.

According to one embodiment of the present invention, if the data privacy button is not triggered, the first data presentation page will continue to be displayed, and the second data presentation page will not be displayed.

According to one embodiment of the present invention, the vaccination data presentation method further includes: receiving a test data from a third party database; according to the test data, generating a scanning result page; and displaying the scanning result page on the electronic device.

The object of the present invention is to provide a vaccination data presentation system which can easily and conveniently provide the vaccination data of individuals and ensure the security of personal information.

To achieve the abovementioned object, a vaccination data presentation system of the present invention includes a transmission module, a storage module and a display interface generation module. The transmission module is used for receiving a vaccination data and an identification data. The storage module is used for storing the vaccination data and the identification data. The display interface generation module is electrically connected to the transmission module and the storage module, and the display interface generation module is used for generating a first data presentation page according to the vaccination data and the identification data and displaying the first data presentation page on an electronic device, wherein the first data presentation page displays the vaccination data and the identification data and includes a data privacy button; if the data privacy button is triggered, the display interface generation module will generate a second data presentation page to replace the first data presentation page, wherein the second data presentation page displays the vaccination data and does not display the identification data.

According to one embodiment of the present invention, the transmission module is further used for receiving a test data from a third party database; the display interface generation module is furthermore used for generating a scanning result page according to the test data and for displaying the scanning result page on the electronic device.

The object of the present invention is to provide a vaccination data authentication server which can easily and conveniently provide the vaccination data of individuals and ensure the security of personal information.

To achieve the abovementioned object, a vaccination data authentication server of the present invention is electrically connected to a third party database and a scanning device. The vaccination data authentication server includes a data transmission module and a data authentication module. The data transmission module is used for receiving a QR code, which is generated via the abovementioned vaccination data presentation method and scanned by the scanning device when displayed on an electronic device. The data authentication module is electrically connected to the data transmission module, wherein the data authentication module is used for determining whether the vaccination data matches a data stored in the third party database to generate a scanning response information.

According to one embodiment of the present invention, the vaccination data authentication server is electrically connected to the electronic device, and the data transmission module sends the scanning response information to the electronic device.

### BRIEF DESCRIPTION OF THE DRAWINGS

FIG. 1 illustrates a system structure drawing of the electronic device, the scanning device, the third party database and the vaccination data authentication server in one embodiment of the present invention.
FIG. 2 illustrates a step flowchart of the vaccination data presentation method in one embodiment of the present invention.
FIG. 3 illustrates a schematic drawing of the first data presentation page displaying the vaccination data and the identification data in one embodiment of the present invention.
FIG. 4 illustrates a schematic drawing of the second data presentation page displaying the QR code but not the vaccination data or identification data in one embodiment of the present invention.
FIG. 5 illustrates a schematic drawing of the vaccination data page in one embodiment of the present invention.
FIG. 6 illustrates a schematic drawing of the test data page in one embodiment of the present invention.
FIG. 7 illustrates a schematic drawing of the test chart page in one embodiment of the present invention.
FIG. 8 illustrates a schematic drawing of the scanning result page in one embodiment of the present invention.
FIG. 9 illustrates a schematic drawing of the content stored in the third party database in one embodiment of the present invention.

### DETAILED DESCRIPTION OF THE PREFERRED EMBODIMENT

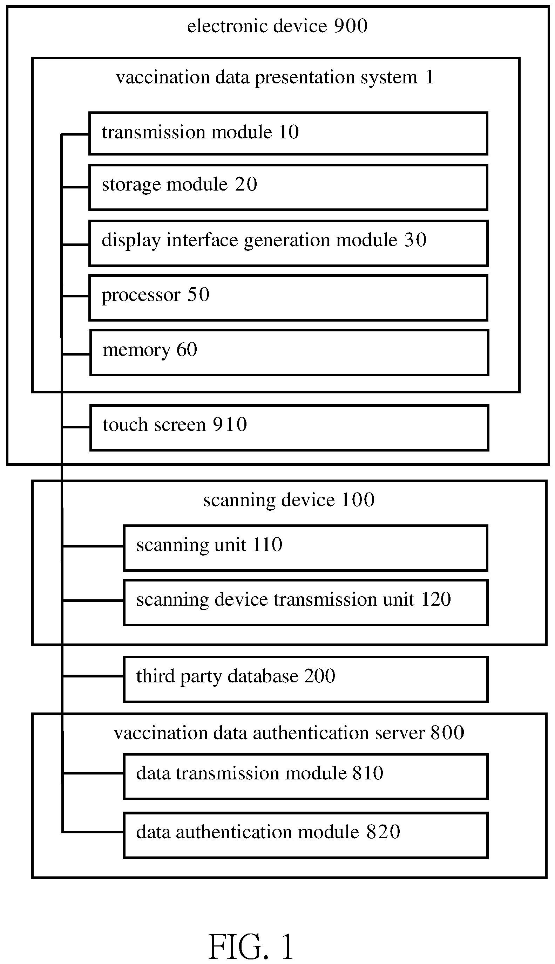

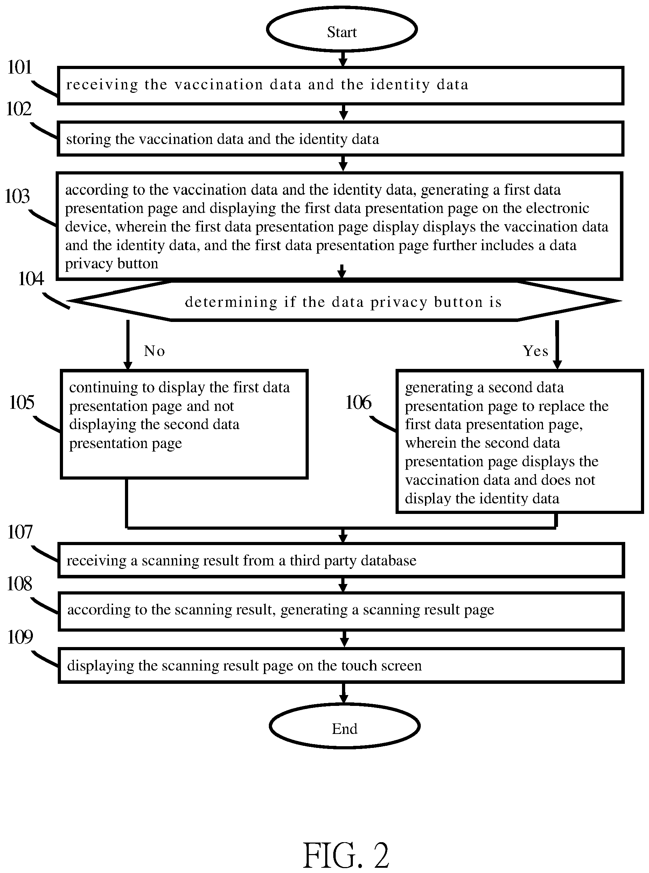

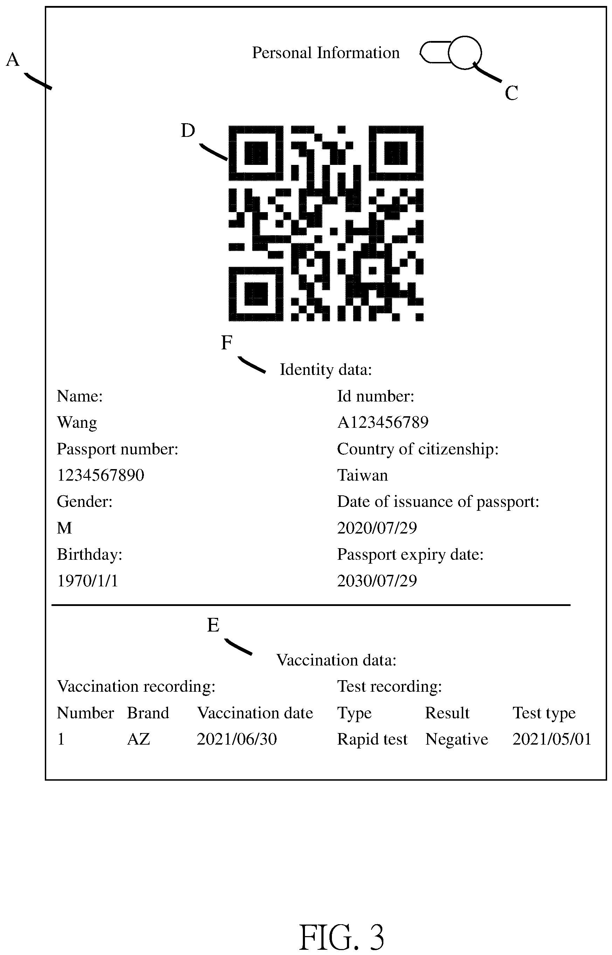

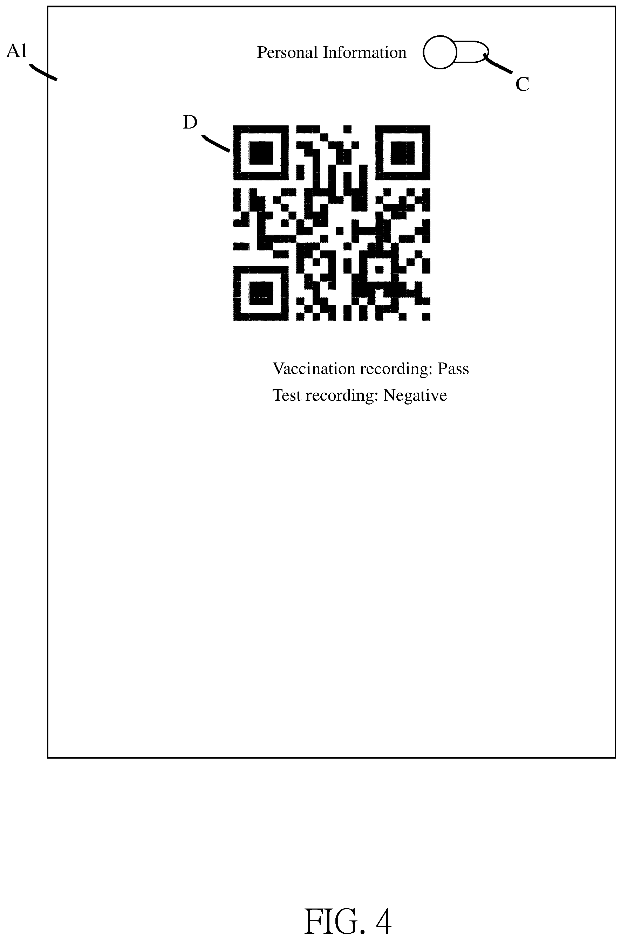

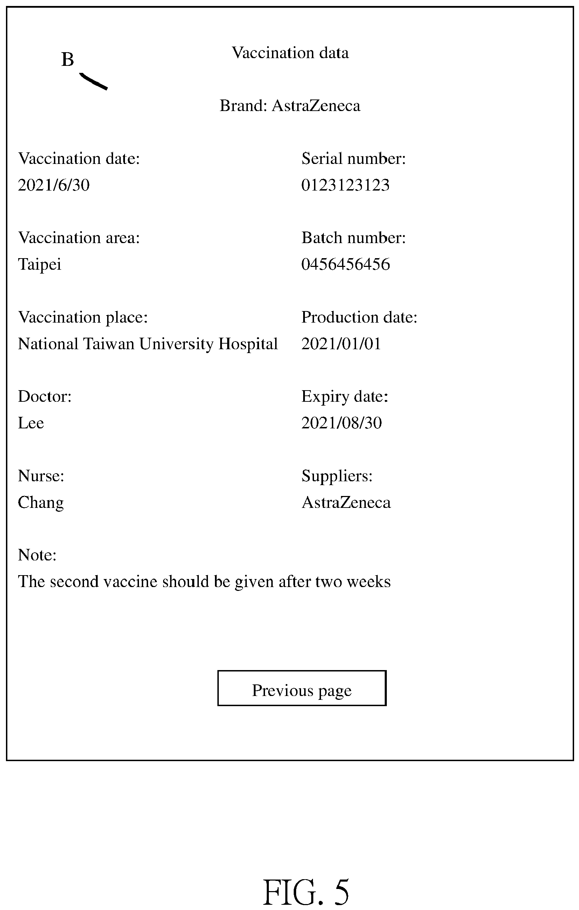

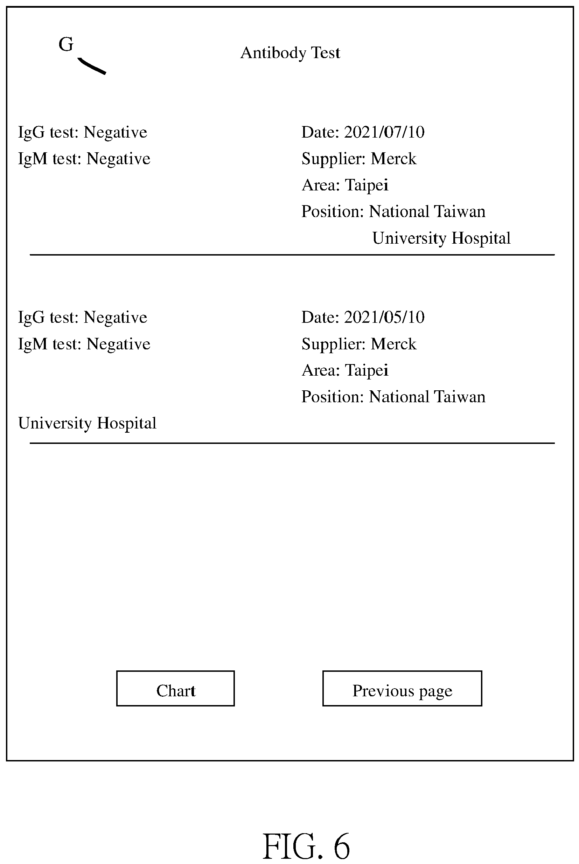

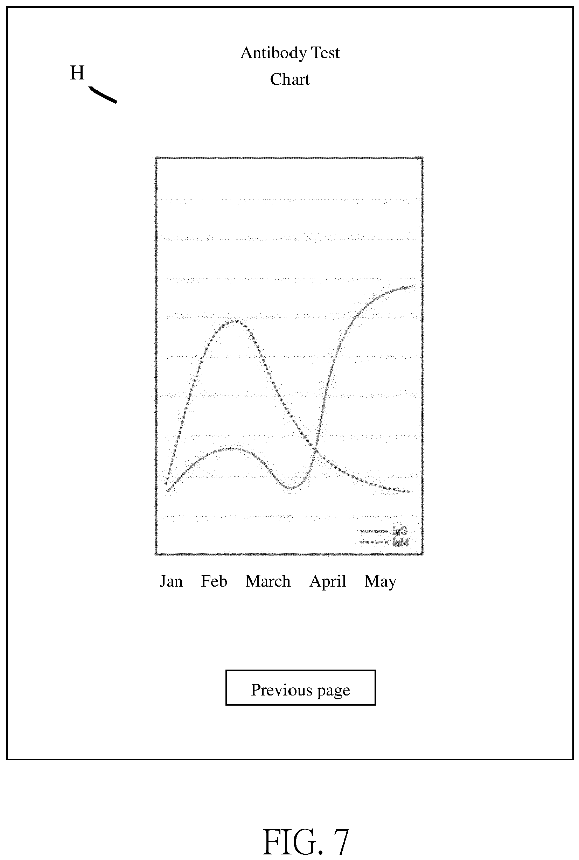

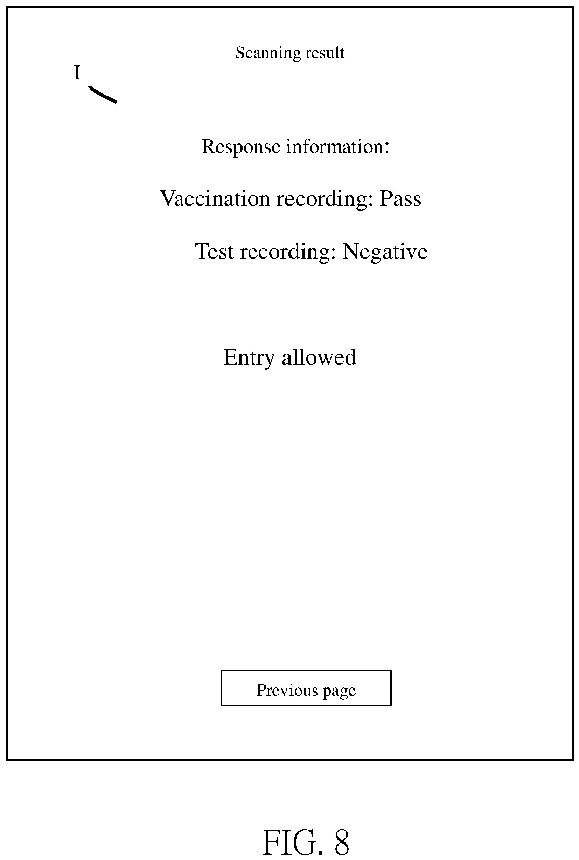

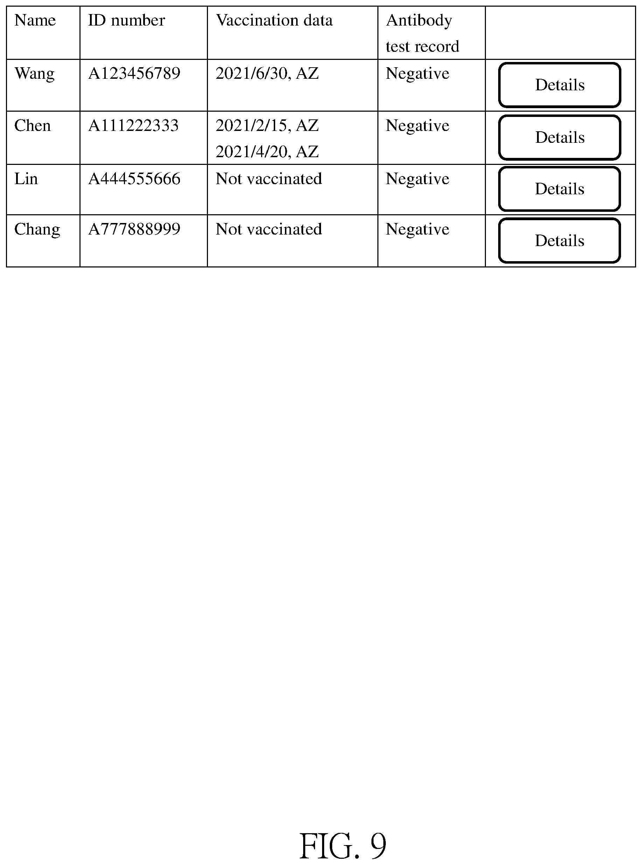

Please refer to FIG. 1 to FIG. 9, which illustrate the vaccination data presentation method and the vaccination data presentation system in one embodiment of the present invention. FIG. 1 illustrates a system structure drawing of the electronic device, the scanning device, the third party database and the vaccination data authentication server in one embodiment of the present invention. FIG. 2 illustrates a step flowchart of the vaccination data presentation method in one embodiment of the present invention. FIG. 3 illustrates a schematic drawing of the first data presentation page displaying the vaccination data and the identification data in one embodiment of the present invention. FIG. 4 illustrates a schematic drawing of the second data presentation page displaying the QR code but not the vaccination data or the identification data in one embodiment of the present invention. FIG. 5 illustrates a schematic drawing of the vaccination data page in one embodiment of the present invention. FIG. 6 illustrates a schematic drawing of the test data page in one embodiment of the present invention. FIG. 7 illustrates a schematic drawing of the test chart page in one embodiment of the present invention. FIG. 8 illustrates a schematic drawing of the scanning result page in one embodiment of the present invention. FIG. 9 illustrates a schematic drawing of the content stored in the third party database in one embodiment of the present invention.

As shown in FIG. 1 and FIG. 9, in one embodiment of the present invention, the electronic device 900 is a smartphone, and the electronic device 900 includes a touch screen 910. The vaccination data presentation system 1 is installed on the electronic device 900, and the vaccination data presentation system 1 can easily and conveniently provide the vaccination data of individuals when scanned with a scanning device 100 and also ensure the security of personal information. The electronic device 900 can be incorporated and work with the scanning device 100, the third party database 200 and the vaccination data authentication server 800. The scanning device 100 is a QR code scanner, which includes a scanning unit 110 and a scanning device transmission unit 120. The scanning device 100 can be applied by an institution that needs to verify the health status of individuals (such as airports, hotels, hospitals, restaurants, and performance venues) for verification of whether the people who enter the institution have been vaccinated for disease resistance. The scanning unit 110 is a common infrared scanning module, laser scanner or other common optical lens for scanning the QR code and reading the content of the QR code. The scanning device transmission unit 120 is a network card, which is electrically connected to the scanning unit 110 and the third party database 200; the scanning device transmission unit 120 is used for sending data to the third party database 200. The third party database 200 is an online disease and vaccine database established by government health agencies for recording the identification data, the vaccination data and the antibody test records of citizens. The identification data includes each individual's name, passport number, gender, birthday, identification (ID) number, country of citizenship, passport date of issue, passport date of expiry and telephone number. The vaccination data includes the vaccine brand, vaccination date, vaccination area, vaccination place, doctor who approved the vaccination, nurse who assisted with the vaccination, serial number, batch number, production date, date of expiry, supplier and notes. The test data includes the type of test conducted, test result, test date, supplier, test area, test position and test value. However, the content of the vaccination data, the identification data and the test data are not limited to the abovementioned description, for the content can be changed according to the using requirement. It is to be known that FIG. 9 shows only the partial content stored in the third party database 200; FIG. 9 does not display all of the abovementioned data stored in the third party database 200. However, the third party database 200 of the present invention comprises a "details" button; if the button is pressed, the third party database 200 can display all of the abovementioned stored data. Further, the type of third party database 200 is not limited to the abovementioned online disease and vaccination database established by government health agencies, for the third party database 200 can also be a cloud-based disease and vaccination database for backing up the data and for allowing the user to upload the user's identification data, vaccination data and antibody test records.

As shown in FIG. 1 and FIG. 3, the vaccination data authentication server 800 is electrically connected to the third party database 200, the scanning device 100 and the electronic device 900. The vaccination data authentication server 800 includes a data transmission module 810 and a data authentication module 820. The data transmission module 810 is used for receiving the QR code D which is read by the scanning device 100 and displayed on the electronic device 900, and the QR code D is generated by the vaccination data presentation method or the vaccination data presentation system 1 of the present invention. The data authentication module 820 is electrically connected to the data transmission module 810 and used for determining whether the vaccination data matches the data stored in the third party database 200 so as to generate a scanning response information and send the scanning response information to the electronic device 900.

The vaccination data presentation system 1 includes a transmission module 10, a storage module 20, a display interface generation module 30, a processor 50 and a memory 60. The transmission module 10 is a chip with a data transmission function and is electrically connected to the touch screen 910, the scanning device 100, the third party database 200 and the vaccination data authentication server 800. The transmission module 10 is used for receiving a vaccination data, an identification data and a test data of a user of the vaccination data presentation system 1, receiving a scanning result sent by the third party database 200, and receiving the scanning response information sent by the vaccination data authentication server 800.

As shown in FIG. 1 and in FIG. 3 to FIG. 8, the storage module 20 is used for storing the data received by the transmission module 10, such as the vaccination data, the identification data and the test data. The display interface generation module 30 is a chip with a data page generation and adjustment function and is electrically connected to the transmission module 10 and the storage module 20. The display interface generation module 30 is used for generating a first data presentation page A, a second data presentation page A1 and a vaccination data page B according to the vaccination data and the identification data, generating a test data page G according to the test data, and generating the scanning result page I according to the scanning result sent by the third party database 200; after the display interface generation module 30 generates the abovementioned pages, the pages can be displayed on the touch screen 910 of the electronic device 900.

As shown in FIG. 1, FIG. 3 and FIG. 4, the first data presentation page A generated by the display interface generation module 30 includes a data privacy button C, a QR code D, a vaccination data E and an identification data F. The content of the QR code D is the identification data, vaccination record, and test record of a user of the vaccination data presentation system 1 and a website address of the third party database 200. The vaccination data E is used for displaying the vaccination record and test record of the user of the vaccination data presentation system 1; the vaccination record includes the vaccination number, vaccine brand and vaccination date; the test record includes the test type, test result and test date. The identification data F displays the name, passport number, gender, birthday, ID number, country of citizenship, passport date of issue, and passport date of expiry of the user of the vaccination data presentation system 1. The data privacy button C is provided for the user to operate; when the data privacy button C is triggered, the display interface generation module 30 will generate the second data presentation page A1 to replace the first data presentation page A. The second data presentation page A1 includes the QR code D, displays the vaccination data and hides the identification data F; thus, the user's personal data can be concealed to ensure the security of personal information; via scanning the QR code D, the result of the comparison of the vaccination record and test record of the user of the vaccination data presentation system 1 can be obtained from the third party database 200.

As shown in FIG. 1 and FIG. 5, the vaccination data page B generated by the display interface generation module 30 displays the vaccination history of the user of the vaccination data presentation system 1, and the vaccination history includes the vaccine brand, vaccination date, vaccination area, vaccination place, doctor who approved the vaccination, nurse who assisted with the vaccination, serial number, batch number, production date, date of expiry, supplier and notes.

As shown in FIG. 1, FIG. 6 to FIG. 8, the test data page G generated by the display interface generation module 30 displays an antibody test data of a disease test of the user of the vaccination data presentation system 1, and the antibody test data includes the type of test that each person has received, test result, test date, supplier, test area and test position. The test data page G includes a test chart page H for displaying a chart generated according to the test value of the test data. A chart button is provided at the bottom of the test data page G; if the user presses the chart button, the touch screen 910 will display the test chart page H. The scanning result page I generated by the display interface generation module 30 displays the vaccination history, test record and review result of the user of the vaccination data presentation system 1 after verification by the third party database 200.

After the display interface generation module 30 generates the first data presentation page A, the second data presentation page A1, the vaccination data page B, the test data page G, the test chart page H and the scanning result page I, the pages will be sent to and displayed on the touch screen 910. The touch screen 910 can also be operated by the user to receive vaccination data, identification data and test data entered by the user. The processor 50 is used for determining if the data privacy button C presented on the touch screen 910 is triggered. If the processor 50 determines that the data privacy button C is triggered, the processor 50 will control the display interface generation module 30 to adjust the content of the first data presentation page A and the second data presentation page A1. The memory 60 is electrically connected to the transmission module 10, the storage module 20, the display interface generation module 30 and the processor 50 for storing software and data for executing the vaccination data presentation method of the present invention.

As shown in FIG. 1 and FIG. 2, the vaccination data presentation method of the present invention is applied to the vaccination data presentation system 1 to allow easy and convenient provision of an individual's vaccination data and to ensure the security of the user's personal information. The vaccination data presentation method is programmed as a software and stored in the memory 60. If a user "Wang" wants to use the vaccination data presentation system 1 to record and display his vaccination record, the user can operate the vaccination data presentation system 1 to execute step 101 of the vaccination data presentation method: receiving the vaccination data and the identification data.

The user can operate the touch screen 910 to enter the user's vaccination data, identification data and test data, and the data are sent to the transmission module 10 of the vaccination data presentation system 1; alternatively, the user can operate the vaccination data presentation system 1 to access the third party database 200 so as to download the vaccination data, the identification data and the test data recorded by the third party database 200 to the vaccination data presentation system 1 via the transmission module 10.

Then the vaccination data presentation system 1 executes step 102 of the vaccination data presentation method: storing the vaccination data and the identification data.

The transmission module 10 sends the received vaccination data, identification data and test data to the storage module 20, and the storage module 20 stores the vaccination data, identification data and test data.

Then the vaccination data presentation system 1 executes step 103 of the vaccination data presentation method: according to the vaccination data and the identification data, generating a first data presentation page and displaying the first data presentation page on the electronic device, wherein the first data presentation page displays the vaccination data and the identification data, and the first data presentation page further includes a data privacy button.

As shown in FIG. 3 to FIG. 7, the display interface generation module 30 generates the first data presentation page A according to the vaccination data, the identification data and the test data stored in the storage module 20 and displays the first data presentation page A on the touch screen 910 of the electronic device. The first data presentation page A further includes a data privacy button C. Further, the display interface generation module 30 generates the vaccination data page B and the test data page G according to the vaccination data, the identification data and the test data stored in the storage module 20, and the user can operate the touch screen 910 to view the first data presentation page A, the vaccination data page B and the test data page G according to the using requirement. The method of alternating among the pages of the present invention is to swipe a finger horizontally on the touch screen 910; however, because this operating method is disclosed in the art of the smartphone, there is no need for further description.

Further, the user can operate the data privacy button C on the first data presentation page A according to the need for the security of personal information. If the user wants to allow the first data presentation page A to display the user's identification data, the user can turn off the data privacy button C (move the data privacy button C to the right); if the user wants to allow the first data presentation page A to hide the user's identification data, the user can trigger the data privacy button C (move the data privacy button C to the left).

Then the vaccination data presentation system 1 executes step 104 of the vaccination data presentation method: determining if the data privacy button is triggered.

The processor 50 determines if the data privacy button C presented on the touch screen 910 is triggered. If the processor 50 determines that the data privacy button C is not triggered, the vaccination data presentation system 1 will execute step 105 of the vaccination data presentation method: continuing to display the first data presentation page and not displaying the second data presentation page.

As shown in FIG. 1 and FIG. 3, the processor 50 will cause the touch screen 910 continue to display the first data presentation page A, and the first data presentation page A includes the QR code D, the vaccination data E and the identification data F. The QR code D provides a hyperlink for an inspector to access the third party database 200 so as to determine whether the vaccination record and test record of the user of the vaccination data presentation system 1 match the content of the third party database 200.

Please return to step 104. As shown in FIG. 1 and FIG. 3, if the processor 50 determines that the data privacy button C is triggered, the vaccination data presentation system 1 will execute step 106 of the vaccination data presentation method: generating a second data presentation page to replace the first data presentation page, wherein the second data presentation page displays the QR code and does not display the vaccination data or identification data.

As shown in FIG. 1 and FIG. 4, the display interface generation module 30 generates the second data presentation page A1, and the second data presentation page A1 displays the QR code D but not the vaccination data E or the identification data F; thus, the personal information of the user can be concealed from others to ensure the security of the user's personal information.

As shown in FIG. 1, FIG. 3, FIG. 4 and FIG. 9, after the user of the vaccination data presentation system 1 "Wang" causes the touch screen 910 to present the first data presentation page A, the user "Wang" can use the vaccination data presentation system 1 to enter the place (such as an airport) where the vaccination and health information needs to be reviewed, and the examiner of the place can hold the scanning device 100 to scan the QR code D on the first data presentation page. After the scanning unit 110 of the scanning device 100 scans the QR code D, the scanning unit 110 will read the content of the QR code D to obtain the identification data, vaccination data, and test result of the user of the vaccination data presentation system 1 and a website address of the third party database 200. The scanning unit 110 then sends the QR code D decoding content to the scanning device transmission unit 120. The scanning device transmission unit 120 accesses the third party database 200 according to the website address and sends the identification data, vaccination data, and test result of the user of the vaccination data presentation system 1 to the third party database 200 so that the third party database 200 can review the vaccination data and the test result of the user in the third party database 200 according to the identification data of the user and then generate a verification result according to the vaccination data and the test result of the user in the third party database 200. The third party database 200 integrates the vaccination data, the test result and the verification result into a scanning result and sends the scanning result to the transmission module 10 and the scanning device transmission unit 120. In the present invention, according to the receiving identification data of Wang (such as the ID number), the third party database 200 reviews the vaccination data (received the AZ vaccine) and the test result (negative) of Wang in the third party database 200, correspondingly generates a scanning result, and sends the scanning result to the transmission module 10 and the scanning device transmission unit 120.

Further, the scanning device 100 reads the QR code and sends the QR code to the data transmission module 810. After the data authentication module 820 determines whether the vaccination data in the content of the QR code matches the data stored in the third party database 200, a scanning response information is generated. The data transmission module 810 sends the scanning response information to the electronic device 900.

Then the vaccination data presentation system 1 executes step 107 of the vaccination data presentation method: receiving a scanning result from a third party database.

The transmission module 10 receives the scanning result sent by the third party database 200 and sends the scanning result to the display interface generation module 30.

Then the vaccination data presentation system 1 executes step 108 of the vaccination data presentation method: according to the scanning result, generating a scanning result page.

As shown in FIG. 1 and FIG. 8, the display interface generation module 30 generates a scanning result page I according to the scanning result, and the scanning result page I displays that the third party database 200 has checked the vaccination data, test record and verification result of the user of the vaccination data presentation system 1.

Finally, the vaccination data presentation system 1 executes step 109 of the vaccination data presentation method: displaying the scanning result page on the touch screen.

The touch screen 910 displays the scanning result page I to inform the user of the scanning result. Similarly, the examiner who operates the scanning device 100 can use the scanning device 100 to review the scanning response information sent by the data transmission module 810 to determine whether the user can enter to restricted location; similarly, the user can display the verification result of the scanning response information, "Entry allowed", to enter the restricted location smoothly.

Via the design of the vaccination data presentation method and the vaccination data presentation system of the present invention, the vaccination data of individuals can be provided easily and conveniently and the security of personal information can be ensured. The vaccination data presentation method and the vaccination data presentation system can work with the scanning device and the third party database to check the user's vaccination history and health status so as to quickly determine whether the user can enter a specific location.

## Claims

1. A vaccination data presentation method, applied to an electronic device (900) for displaying a vaccination data (E), the vaccination data presentation method comprising:
receiving the vaccination data (E) and an identification data (F);
storing the vaccination data (E) and the identification data (F); and
according to the vaccination data (E) and the identification data (F), generating a first data presentation page (A) and displaying the first data presentation page (A) on the electronic device (900), wherein the first data presentation page (A) displays the vaccination data (E) and the identification data (F), and the first data presentation page (A) furthermore comprises a data privacy button (C); if the data privacy button (C) is triggered, generating a second data presentation page (A1) to replace the first data presentation page (A), wherein the second data presentation page (A1) displays the vaccination data (E) and does not display the identification data (F).

2. The vaccination data presentation method as claimed in Claim 1, wherein the first data presentation page (A) and the second data presentation page (A1) both comprise a QR code (D).

3. The vaccination data presentation method as claimed in Claim 2, wherein if the data privacy button (C) is not triggered, the first data presentation page (A) will continue to be displayed, and the second data presentation page (A1) will not be displayed.

4. The vaccination data presentation method as claimed in Claim 2, further comprising: receiving a test data from a third party database (200); according to the test data, generating a scanning result page (I); and displaying the scanning result page (I) on the electronic device (900).

5. A vaccination data presentation system (1), comprising:
a transmission module (10), for receiving a vaccination data (E) and an identification data (F);
a storage module (20), for storing the vaccination data (E) and the identification data (F); and
a display interface generation module (30), electrically connected to the transmission module (10) and the storage module (20), for generating a first data presentation page (A) according to the vaccination data (E) and the identification data (F) and displaying the first data presentation page (A) on an electronic device (900), wherein the first data presentation page (A) displays the vaccination data (E) and the identification data (F) and the first data presentation page (A) comprises a data privacy button (C); if the data privacy button (C) is triggered, the display interface generation module (30) will generate a second data presentation page (A1) to replace the first data presentation page (A), wherein the second data presentation page (A1) displays the vaccination data (E) and does not display the identification data (F).

6. The vaccination data presentation system (1) as claimed in Claim 5, wherein the first data presentation page (A) and the second data presentation page (A1) both comprise a QR code (D).

7. The vaccination data presentation system (1) as claimed in Claim 6, wherein if the data privacy button (C) is not triggered, the first data presentation page (A) will continue to be displayed and the second data presentation page (A1) will not be displayed.

8. The vaccination data presentation system (1) as claimed in Claim 6, wherein the transmission module (10) is further used for receiving a test data from a third party database (200); the display interface generation module (30) is furthermore used for generating a scanning result page (I) according to the test data and for displaying the scanning result page (I) on the electronic device (900).

9. A vaccination data authentication server (800), electrically connected to a third party database (200) and a scanning device (100), the vaccination data authentication server (800) comprising:
a data transmission module (810), for receiving the QR code (D) which is generated via the vaccination data presentation method as claimed in Claim 2 and scanned by the scanning device (100) when displayed on an electronic device (900); and
a data authentication module (820), electrically connected to the data transmission module (810), wherein the data authentication module (820) is used for determining whether the vaccination data (E) matches a data stored in the third party database (200) so as to generate a scanning response information.

10. The vaccination data authentication server (800) as claimed in Claim 9, wherein the vaccination data authentication server (800) is electrically connected to the electronic device (900) and the data transmission module (810) sends the scanning response information to the electronic device (900).
